# EUROPEAN PATENT APPLICATION

(11) **EP 1 705 246 A1**
(43) Date of publication of application: **27.09.2006**
(21) Application number: 05398004.1
(22) Date of filing: 24.03.2005
(51) Int. Cl.: C12N 9/96, C12N 1/04, A61K 31/70

(54) **Glycerophosphoinositol as a stabilizer and / or preservative of biological materials**

(71) Applicant: IBET - Instituto de Biologia Experimental e Tecnologica, 2781-901 Oeiras (PT)
(72) Inventor: Dias dos Santos, Maria Helena, 2775-713 Carcavelos (PT); Lamosa António, Pedro Miguel, 2750 Cascais (PT); Cafeira Gonçalves, Luis Pedro, 100-196 Lisboa (PT); Viseu Rodrigues, Marta, 1750-247 Lisboa (PT); Hammond Raven, Neil David, Marlborough, Wiltshire SN8 3TY (GB)

(57) **Abstract**

The present invention consists in the utilization of glycerophosphoinositols, like 1-*myo*-inositol-1-phosphoglycerol, or mixtures thereof alone or as constituents in a suitable formulation as protectors and/or stabilisers of proteins, enzymes, antibodies, DNA or RNA molecules, biological membranes, lipids, lipossomes, lipid related substances or other cellular components and biomaterials against general stress.

## Description

### FIELD OF THE INVENTION

The present invention concerns the use of glycerophosphoinositol, alone or as constituent of a formulation to protect and/or stabilise proteins, nucleic acids, lipids, lipid like substances or other cellular components and biomaterials against general stress, namely caused by heat, high osmolarity, free-radicals, desiccation, freeze-drying, and repetitive use.

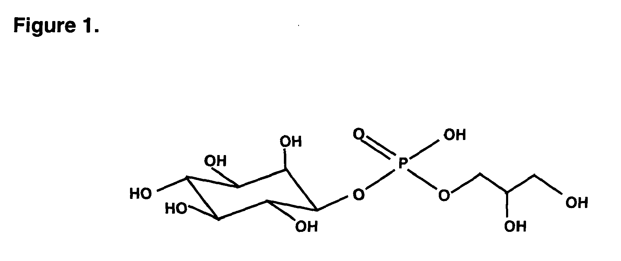

The said compound obeys to the general formula represented in Figure 1, which is intended to depict glycerophosphoinositol in all its possible stereoisomeric forms.

### DESCRIPTION OF THE INVENTION AND STATE OF THE ART

The accumulation of low-molecular mass organic solutes such as, trehalose, polyols or ectoines, is a prerequisite for osmotic adjustment of many mesophilic microorganisms. However, very unusual solutes namely, di-*myo-*inositol-phosphate, di-mannosyl-di-*myo*-inositol-phosphate, diglycerol phosphate, mannosylglycerate, and mannosylglyceramide, have been identified in thermophilic and hyperthermophilic microorganisms and the intracellular content of these solutes increases in response to stress conditions, such as high osmolarity or high temperature.

Mannosylglycerate and diglycerol phosphate have been studied to a greater extent and have been shown to protect enzymes and proteins *in vitro* better than compatible solutes from mesophiles [1-3]. Moreover, the application of compatible solutes from thermophilic or hyperthermophilic organisms as stabilising agents of biomaterials has been disclosed in several patent applications [4-6].

We have discovered a novel compatible solute in the hyperthermophilic bacterium *Aquifex pyrophilus,* and in the hyperthermophilic archaeon *Archaeoglobus fulgidus.* Both organisms share, approximately, the same growth conditions (optimal growth around 80°C and 2% NaCl). When subjected to a combination of salt and temperature stresses both organisms accumulate the novel compound. In the case of *Aq. pyrophilus* this accumulation, as a response to a combination of temperature and salt stress, can be massive reaching more than 2 µmol/mg of protein, and becoming the major accumulating solute. After extraction, purification, and full spectroscopic characterization by Nuclear Magnetic Resonance, we have determined the molecular structure of this, to date, unidentified compound as 1-phosphoglycerol-1-*myo*-inositol or glycerophosphoinositol (GPI).

It is interesting to note that the molecular structure of this compatible solute resembles a chimera between di-*myo*-inositol-1,1'-phosphate (DIP) and di-glycerol-1,1'-phosphate (DGP). While DIP is a widely distributed solute among hyperthermophiles responding strongly to supraoptimal growth temperatures [7], DGP is a strong protein thermal stabilizer [3].

Because of the thermal protection abilities of DGP and DIP, industrial utilizations of both solutes are protected under European patent applications [5-6]. The thermophilic origin of the novel solute, its pattern of accumulation in response to combined temperature and salt stress, and its structural resemblance to DIP and DGP leads us to propose that this novel solute has stabilising properties as good or superior to those already demonstrated for DIP and DGP. In this respect, it can serve as a stabiliser in various commercial, industrial, medical, pharmaceutical, diagnostic, cosmetic, or academic research applications.

Although, glycerophosphoinositol had generically been previously envisioned as a possible cryopreservative in long term storage of tissues from animal or vegetable origin [8], its use as a highly efficient protector of enzymes, proteins, lipid vesicles or microbial cell lines against heat salt stress, desiccation, transport or routine usage, had never been disclosed.

The enhanced protein stability rendered by certain low-molecular mass organic solutes allows enzymes to function under more severe conditions of temperature, pressure, ionic strength, pH, presence of detergents or organic solvents. One of the priorities of modern biotechnology is to obtain stable enzymes or agents that stabilise those enzymes against thermal or chemical denaturation. The ability of some compatible solutes to stabilise enzymes is, therefore, of great importance to modern biotechnology. This point is obviously extended to all proteins that are used or can be used in processes where their stability is an issue, since all proteins either with or without enzymatic activity share the same overall basic elements of structure and may be protected against denaturation or inactivation through the same general mechanisms or processes.

It must also be stressed that compatible solutes protect proteins, cell membranes, and lipossomes from the deleterious effects of desiccation, and possess strong moistening properties. The preservation of desiccated or lyophilized cell components and biomaterials has many applications in medicine, pharmaceutical industry, cosmetic industry, food industry, and scientific research. In spite of the great importance of desiccation and freezing in the conservation of biological samples, denaturation of proteins, disruption of lipossomes, or degradation of nucleic acids inevitably takes place during utilization or transport, and could be prevented or diminished by the use of low molecular mass stabilisers.

Also, the stability of nucleic acid molecules, like DNA, or RNA, can be improved by the addition of compatible solutes from hyperthermophiles, as described for ectoines [9], and their use in several applications in medicine, pharmaceutical industry, or scientific research can be envisioned.

### BRIEF DESCRIPTION OF THE DRAWING

### Figure 1.

Depicts the generic chemical structure of 1-phosphoglycerol-1-*myo*-inositol in all its possible stereoisomeric forms. The figure is intended to represent all inositol and glycerol conformations.

### REFERENCES

[1] Ramos, A., N. D. H. Raven, R. J. Sharp, S. Bartolucci, M. Rossi, R. Cannio, J. Lebbink, J. van der Oost, W. M. de Vos and H. Santos. 1997. Stabilisation of enzymes against thermal stress and freeze-drying by mannosylglycerate. Appl. Environ. Microbiol. 63:4020-4025.
[2] Borges, N., A. Ramos, N. D. H. Raven, R. J. Sharp, and H. Santos. 2002. Comparative study of the thermostabilising properties of mannosylglycerate and other compatible solutes on model enzymes. Extremophiles 6:209-216.
[3] Lamosa, P., A. Burke, R. Peist, R. Huber, M. Liu, G. Silva, C. Rodrigues-Pousada, J. LeGall, C. Maycock, and H. Santos. 2000. Thermostabilisation of proteins by diglycerol phosphate, a new compatible solute from the hyperthermophile Archaeoglobus fulgidus. Appl. Environ. Microbiol. 66:1974-1979.
[4] Santos, H., A. Ramos, M. S. da Costa. Thermostabilisation, osmoprotection, and protection against desiccation of enzymes, cell components and cells by mannosylglycerate. European Patent no. 97670002.1
[5] Santos, H., P. Lamosa, C. Maycock, and A. Burke. 1999. Thermostabilisation, osmoprotection, and protection against desiccation of enzymes, cell components and cells by di-glycerol-phosphate. European Patent no. 98670002.9
**[6]** Santos, H., L. O. Martins, L. S. Carreto, and M. S. da Costa. 1996. Utilização de fosfato de di-manosil-di-mio-inositol e de fosfato de 1,3'- di-mio-inositol na termo-estabilisação, osmoprotecção e protecção contra a desidratação de components celulares e células. Portuguese patent no. 101813.
[7] Santos. H., and M. S. da Costa. 2001. Organic solutes from thermophiles and hyperthermophiles. Methods Enzymol. 334:302-315.
[8] Glonek, T., and J. V. Greiner. 1989. Method and composition for cryopreservation of tissue. European Patent Application nº 89114343.0.
[9] Malin G, lakobashvili R, and Lapidot A (1999) Effect of tetrahydropyrimidine derivatives on protein-nucleic acids interaction. Type II restriction endonucleases as a model system. J. Biol. Chem. 274:6920-6929.

## Claims

1. The utilization of glycerophosphoinositols, namely 1-phosphoglycerol-1-myo-inositol, wherein the compound is in any of its possible stereoisomeric forms, alone or as a constituent in a suitable formulation to protect and/or stabilise proteins, enzymes, nucleic acids, lipids, lipid related substances, or other cellular components and biomaterials against general stress.

2. The utilization of glycerophosphoinositols according to claim 1 wherein the *myo*-inositol residue is replaced by any inositol isomeric form, namely, *scyllo-*inositol, *chiro*-inositol, or *epi*-inositol.

3. The utilization of glycerophosphoinositols according to claims 1 and 2 wherein the inositol residue is linked to the phosphate moiety by any of its six possible positions, namely positions 1, 2, 3, 4, 5, or 6.

4. The utilization of glycerophosphoinositols according to claims 1 to 3 wherein the glyceryl residue is in the D or L configuration.

5. The utilization of glycerophosphoinositols according to claims 1 to 4 wherein the glyceryl residue is linked to the phosphate moiety by any of its three possible positions, namely positions 1, 2, or 3.

6. The utilization of glycerophosphoinositols or mixtures thereof according to claims 1 to 5 wherein to protect enzymes or other proteins against denaturation induced by purification, transport, storage and/or repetitive use.

7. The utilization of glycerophosphoinositols or mixtures thereof according to claims 1 to 6 wherein as a protector of the activity of polymerase chain reaction (PCR) enzymes for clinical, biological and industrial purposes during storage, as well as the high-temperature recycling of the enzymes.

8. The utilization of glycerophosphoinositols or mixtures thereof according to claims 1 to 6 wherein as a stabiliser of enzymes or other proteins during lyophilization, desiccation or freeze-drying, transport, and storage at low temperatures.

9. The utilization of glycerophosphoinositols or mixtures thereof according to claims 1 to 6 wherein as a stabilising agent during the manufacture, transport, storage and assays using test kit enzymes, for diagnostic, biological and industrial purposes.

10. The utilization of glycerophosphoinositols or mixtures thereof according to claims 1 to 6 wherein to stabilise enzymes or other proteins during their routine utilization for clinical, biological and industrial purposes.

11. The utilization of glycerophosphoinositols or mixtures thereof according to claims 1 to 5 wherein for the protection or stabilisation of antibodies for clinical, biological, research, and industrial purposes.

12. The utilization of glycerophosphoinositols or mixtures thereof according to claims 1 to 5 wherein for the protection or stabilisation of vaccines of proteic or non-proteic nature for research, clinical, or industrial purposes.

13. The utilization of glycerophosphoinositols or mixtures thereof according to claims 1 to 5 and 11 to 12 wherein for the protection or stabilisation of vaccines of proteic or non-proteic nature or antibodies for research, clinical, or industrial purposes, during transport.

14. The utilization of glycerophosphoinositols or mixtures thereof according to claims 1 to 5 wherein for the protection of biomaterials against stress such as desiccation and lyophilization of cell membranes, liposomes, liposome-containing cosmetics or lipid related substances.

15. The utilization of glycerophosphoinositols or mixtures thereof according to claims 1 to 5 wherein for the structural protection or stabilisation of DNA or RNA molecules.

16. The utilization of glycerophosphoinositols or mixtures thereof according to claims 1 to 5 and 15 wherein for the protection, stabilisation or improved performance of DNA or RNA microarrays.

17. The utilization of glycerophosphoinositols or mixtures thereof according to claims 1 to 5 and 15 to 16 wherein for the protection or stabilisation of DNA or RNA microarrays during transport or storage.

18. The utilization of glycerophosphoinositols or mixtures thereof according to claims 1 to 5 wherein as an additive to cosmetics to improve moistening properties, stabilise compounds or lipossomes or as a suppressor of free radicals.

19. The utilization of glycerophosphoinositols or mixtures thereof according to claims 1 to 18 wherein the compound is a constituent in a suitable formulation to protect and/or stabilise proteins, enzymes, nucleic acids, lipids, lipid related substances, or other cellular components and biomaterials against general stress.
